# EUROPÄISCHE PATENTSCHRIFT

(11) **EP 0 912 498 B1**
(45) Veröffentlichungstag und Bekanntmachung des Hinweises auf die Patenterteilung: **12.05.2004**
(21) Anmeldenummer: 97929274.5
(22) Anmeldetag: 24.06.1997
(51) Int. Cl.: C07C 233/65, C07C 231/02, A61K 31/165

(54) **4(4-OXOCYCLOHEXYL) BENZAMIDE ALS ZWISCHENPRODUKTE FÜR ARZNEIMITTEL**
4(4-OXOCYCLOHEXYL) BENZAMIDES AS INTERMEDIATE PRODUCTS FOR MEDICAMENTS
4(4-OXOCYCLOHEXYLE) BENZAMIDES S'UTILISANT COMME PRODUITS INTERMEDIAIRES POUR DES MEDICAMENTS

(30) Priorität: 03.07.1996 DE 19626771
(43) Veröffentlichungstag der Anmeldung: 06.05.1999
(73) Patentinhaber: MERCK PATENT GmbH, 64271 Darmstadt (DE)
(72) Erfinder: WÄCHTLER, Andreas, D-72076 Tübingen (DE); STERN, Margit, D-64287 Darmstadt (DE); REIFFENRATH, Volker, D-64380 Ro dorf (DE)
(86) Internationale Anmeldenummer: PCT/EP1997/003299
(87) Internationale Veröffentlichungsnummer: WO 1998/001420

(56) Entgegenhaltungen:
- EP-A- 0 331 933
- DE-A- 4 239 150

## Beschreibung

Die Erfindung betrifft Benzamide der Formel I 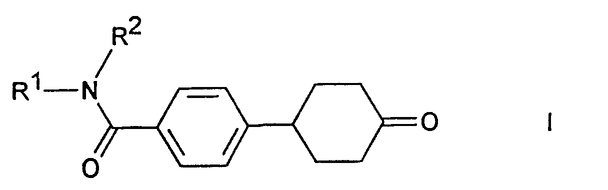 worin
- R¹, R²: jeweils unabhängig voneinander Alkyl mit 1-6 C-Atomen,
- R¹ und R²: zusammen auch Alkylen
bedeuten,
sowie deren Salze.

Der Erfindung lag die Aufgabe zugrunde, neue Verbindungen zu finden, die insbesondere als Zwischenprodukte bei der Synthese von Arzneimitteln verwendet werden können.

Es wurde gefunden, daß die Verbindungen der Formel I und ihre Salze wichtige Zwischenprodukte zur Herstellung von Arzneimitteln darstellen.

DE-A-4239150 beschreibt O-Acyl-4-phenyl cyclohexanole und Zwischenprodukte dafür, die zur Behandlung von Artherosklerose dienen.

Gegenstand der Erfindung sind die Benzamidderivate der Formel I und ihre Salze.

Vor- und nachstehend haben die Reste R¹ und R² die bei den Formeln I bis III angegebenen Bedeutungen, falls nicht ausdrücklich etwas anderes angegeben ist.

In den vorstehenden Formeln ist Alkyl vorzugsweise unverzweigt und hat 1, 2, 3, 4, 5 oder 6 C-Atome, vorzugsweise 1, 2, 3, 4 oder 5 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, neoPentyl oder Isopentyl.

Alkylen bedeutet z.B. Methylen, vorzugsweise Ethylen, Propylen, Butylen oder Pentylen.

Gegenstand der Erfindung ist ferner ein Verfahren zur Herstellung von Benzamiden der Formel I nach Anspruch 1 sowie von deren Salzen, dadurch gekennzeichnet, daß man eine Verbindung der Formel II 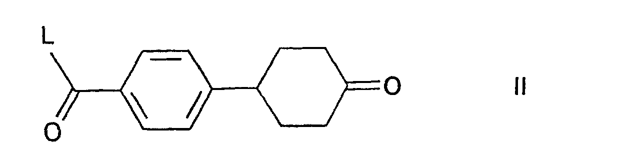 worin
- L: Cl, Br, OH oder eine reaktionsfähige veresterte OH-Gruppe bedeutet,
mit einer Verbindung der Formel III

HNR¹R² III

oder einem ihrer Salze,
worin R¹ und R² die angegebenen Bedeutungen haben,
umsetzt,
und/oder
daß man eine Base der Formel I durch Behandeln mit einer Säure in eines ihrer Salze umwandelt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

In den Verbindungen der Formeln II bedeutet der Rest L vorzugsweise Cl oder Br; er kann jedoch auch I, OH oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl-, p-Tolylsulfonyloxy, 1- oder 2-Naphthalinsulfonyloxy) bedeuten.

Die Umsetzung der Verbindungen der Formeln II mit Verbindungen der Formeln III, verläuft nach Methoden, wie sie für die Acylierung von Aminen aus der Literatur bekannt sind. Man kann auch ohne Gegenwart eines Lösungsmittels die Komponenten miteinander verschmelzen, gegebenenfalls im geschlossenen Rohr oder im Autoklaven.

Die Verbindungen der Formel III können auch in Form ihrer Salze eingesetzt werden, wie dies beispielsweise beschrieben ist von Davidson et al., Synthetic Commun. 20, 727-732 (1990).

Es ist aber auch möglich, die Verbindungen in Gegenwart eines inerten Lösungsmittels umzusetzen.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Nitroverbindungen wie Nitromethan oder Nitrobenzol; Ester wie Ethylacetat, gegebenenfalls auch Gemische der genannten Lösungsmittel untereinander oder Gemische mit Wasser.

Der Zusatz eines säurebindenden Mittels, beispielsweise eines Alkalioder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein. Die Reaktionszeit liegt je nach den angewendeten Bedingungen zwischen einigen Minuten und 14 Tagen, die Reaktionstemperatur zwischen -20 und 100°, normalerweise zwischen -10 und 40°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist weiterhin die Verwendung der Verbindungen der Formel I als Zwischenprodukte zur Synthese von Arzneimitteln.

Gegenstand der Erfindung ist auch die Verwendung der Verbindungen der Formel I nach Anspruch 1 in Reduktionsreaktionen, dadurch gekennzeichnet, daß die Reaktion der Verbindungen der Formel I mit komplexen Metallhydriden erfolgt.

Die Reduktion der erfindungsgemäßen Ketoamide kann z.B. mit Lithiumaluminiumhydrid analog der Methode von Deslongchamps et al., Can. J. Chem., 53, 3613-3619 (1975), erfolgen.

Ferner können als Reduktionsmittel z.B. NaBH₄ oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich z.B. die oben erwähnten inerten Lösungsmittel.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel.

### Beispiel 1

Eine wässerige Lösung (15 %) aus 3 Mol Dimethylamin wird mit 4-(4-Oxocyclohexyl)-benzoesäurechlorid [erhältlich durch Umsetzung von 0,75 Mol (130,7 g) 4-Phenylcyclohexanon mit 1 Mol Oxalylchlorid und 2 Mol AlCl₃ in Dichlormethan und anschließender Hydrolyse mit Eiswasser/HCl] versetzt und 2 Stunden bei 0 bis 5° gerührt. Man arbeitet wie üblich auf und erhält 125,2 g N,N-Dimethyl-4-(4-oxocyclohexyl)-benzamid, F. 118°; Ausbeute: 68 % bezogen auf 4-Phenylcyclohexanon.

### Beispiel 2

Äquivalente Mengen an (4-Oxocyclohexyl)-benzoesäurechlorid und Dimethylamin-hydrochlorid werden in Dichlormethan gelöst. Anschließend wird eine äquivalente Menge an Triethylamin zugegeben und 3 Stunden gerührt.
Nach üblicher Aufarbeitung erhält man N,N-Dimethyl-4-(4-oxocyclohexyl)-benzamid, F. 118°.

## Patentansprüche

1. Benzamide der Formel I 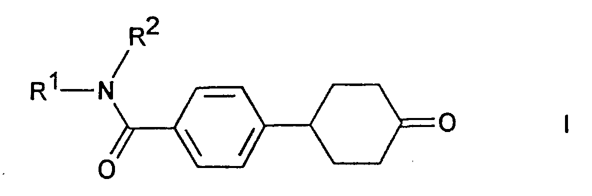 worin
R¹, R² jeweils unabhängig voneinander Alkyl mit 1-6 C-Atomen,
R¹ und R² zusammen auch Alkylen
bedeuten,
sowie deren Salze.

2. N,N-Dimethyl-4-(4-oxocyclohexyl)-benzamid und dessen Salze gemäß Anspruch 1.

3. Verfahren zur Herstellung von Benzamiden der Formel I nach Anspruch 1 sowie von deren Salzen, **dadurch gekennzeichnet, daß** man eine Verbindung der Formel II 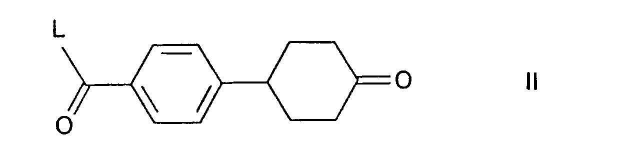 worin
L Cl, Br, OH oder eine reaktionsfähige veresterte OH-Gruppe bedeutet,
mit einer Verbindung der Formel III
HNR¹R² III
oder einem ihrer Salze,
worin R¹ und R² die angegebenen Bedeutungen haben,
umsetzt,
und/oder
daß man eine Base der Formel I durch Behandeln mit einer Säure in eines ihrer Salze umwandelt.

4. Verwendung der Verbindungen der Formel I nach Anspruch 1 als Zwischenprodukte zur Synthese von Arzneimitteln.

5. Verwendung der Verbindungen der Formel I nach Anspruch 1 in Reduktionsreaktionen, **dadurch gekennzeichnet, daß** die Reaktion der Verbindungen der Formel I mit komplexen Metallhydriden erfolgt.

## Claims

1. Benzamides of the formula I 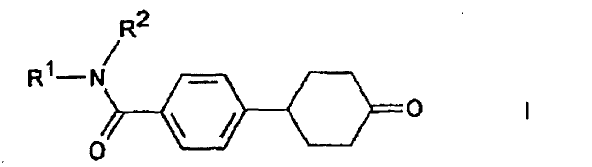 in which
R¹, R² each, independently of one another, denote alkyl having 1-6 C atoms,
R¹ and R² together also denote alkylene,
and salts thereof.

2. N,N-Dimethyl-4-(4-oxocyclohexyl)benzamide and salts thereof according to Claim 1.

3. Process for the preparation of benzamides of the formula I according to Claim 1 and of salts thereof, **characterised in that** a compound of the formula II 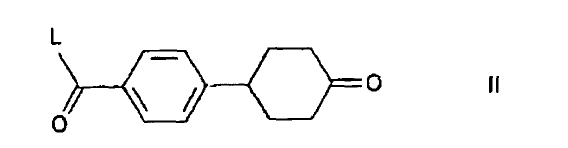 in which
L denotes Cl, Br, OH or a reactive esterified OH group,
is reacted with a compound of the formula III
HNR¹R² III
or one of its salts,
in which R¹ and R² have the meanings indicated,
and/or
**in that** a base of the formula I is converted into one of its salts by treatment with an acid.

4. Use of the compounds of the formula I according to Claim 1 as intermediates for the synthesis of medicaments.

5. Use of the compounds of the formula I according to Claim 1 in reduction reactions, **characterised in that** the reaction of the compounds of the formula I is carried out with complex metal hydrides.

## Revendications

1. Benzamides de la formule I 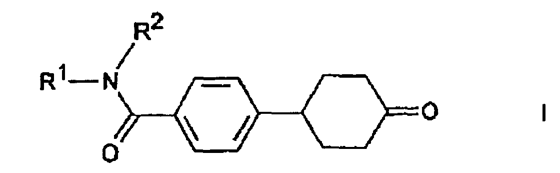 dans lesquels
R¹, R² chacun, indépendamment de l'autre, représente un alkyl ayant 1-6 atomes C,
R¹ et R² représentent ensemble également un alkylène,
et des sels de ceux-ci.

2. N,N-diméthyl-4-(4-oxocyclohexyl)benzamide et des sels de celui-ci selon la revendication 1.

3. Procédé pour la préparation de benzamides de la formule I selon la revendication 1 et des sels de ceux-ci, **caractérisé en ce qu'**un composé de la formule II 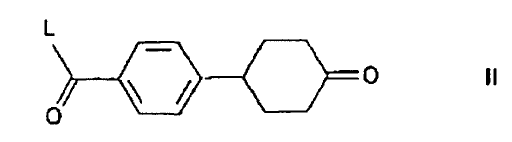 dans lequel
L représente CI, Br, OH ou un groupe OH estérifié par réactif,
est amené à réagir avec un composé de la formule III
HNR¹R² III
ou avec un de ses sels,
dans lequel R¹ et R² ont la signification indiquée,
et/ou
**en ce qu'**une base de la formule I est convertie selon l'un de ses sels par traitement avec un acide.

4. Utilisation des composés de la formule I selon la revendication 1 en tant qu'intermédiaires pour la synthèse de médicaments.

5. Utilisation des composés de la formule I selon la revendication 1 dans des réactions de réduction, **caractérisée en ce que** la réaction des composés de la formule I est mise en oeuvre avec de hydrures métalliques complexes.
